# EUROPEAN PATENT APPLICATION

(11) **EP 4 692 329 A1**
(43) Date of publication of application: **11.02.2026**
(21) Application number: 24785316.1
(22) Date of filing: 05.04.2024
(51) Int. Cl.: C12N 5/079

(54) **COMPOSITION FOR INDUCING DIRECT DIFFERENTIATION OF STEM CELLS INTO CORNEAL ENDOTHELIAL CELLS AND METHOD FOR INDUCING DIRECT DIFFERENTIATION USING SAME**

(30) Priority: 06.04.2023 KR 20230045488
(71) Applicant: The Asan Foundation, Seoul 05505 (KR); UNIVERSITY OF ULSAN FOUNDATION FOR INDUSTRY COOPERATION, Nam-gu Ulsan 44610 (KR)
(72) Inventor: LEE, Hun, Seoul 05505 (KR); YE, Eun Ah, Seoul 05505 (KR)
(74) Representative: AWA Sweden AB
(86) International application number: PCT/KR2024/004522
(87) International publication number: WO 2024/210619

(57) **Abstract**

The present invention relates to a composition for inducing the differentiation of stem cells into corneal endothelial cells and a method for inducing differentiation using same. By using the differentiation composition according to one aspect of the present invention, the time and cost required for differentiation from stem cells to corneal endothelial cells can be reduced, with the consequent increase of differentiation efficiency. The corneal endothelial cells differentiated by the method exhibit gene expression patterns specific to corneal endothelial cells similar to those of human-derived corneal endothelial cells and thus can be widely applied as an alternative cell source for corneal endothelial cell transplantation.

## Description

### Technical Field

The present disclosure relates to a composition for inducing direct differentiation of stem cells into corneal endothelial cells and a method of inducing direct differentiation using the same.

### Background Art

The cornea is the outermost structure of the eye and one of the main organs responsible for refracting light. The cornea consists of six layers: corneal epithelium, Bowman's layer, corneal stroma (corneal substance), Dua's layer, Descemet's membrane, and corneal endothelium.

The corneal endothelium is a monolayer of hexagonal cells located on the posterior surface of the cornea and functions as a physiological ion pump. At birth, the average density of human corneal endothelial cells (CECs) is known to be about 5,000 cells/mm². However, because these cells have limited mitotic potential, the total number of cells decreases with age.

When corneal endothelial cells (CECs) are damaged, the cells repair through cell expansion and migration rather than mitosis, which causes the cornea to swell to several times its normal thickness, become opaque, and lose its function. Such damage is permanent.

Currently, established treatment for corneal endothelial cell damage is corneal transplantation. Recovery may be achieved by transplanting donor tissue via penetrating keratoplasty (PK) or lamellar keratoplasty.

However, because the supply of donor corneas is severely limited, there is an urgent need to develop a method capable of efficiently inducing differentiation of stem cells into corneal endothelial cells to overcome this limitation.

In view of the foregoing, the present inventors have developed a method of directly differentiating stem cells into corneal endothelial cells, wherein the corneal endothelial cells differentiated by the method exhibit an expression pattern of corneal endothelial cell-specific genes that is highly similar to that of human-derived corneal endothelial cells, and thus may be used as an alternative cell source for corneal endothelial cell transplantation. Moreover, compared to conventional methods for differentiating into corneal endothelial cells, the method offers the advantage of reducing the time and cost required for differentiation.

### Disclosure of Invention

### Technical Problem

One aspect provides a method of differentiating stem cells into corneal endothelial cells, the method including culturing the stem cells in a culture medium containing a Rho-associated protein kinase (ROCK) inhibitor to differentiate the stem cells into corneal endothelial cells (CECs).

Another aspect provides a culture medium composition for inducing direct differentiation of stem cells into corneal endothelial cells, the composition containing a Rho-associated protein kinase (ROCK) inhibitor.

Another aspect provides a kit for inducing direct differentiation of stem cells into corneal endothelial cells, the kit including: a composition containing a Rho-associated protein kinase (ROCK) inhibitor; and a culture vessel modified with an extracellular matrix (ECM).

### Solution to Problem

An aspect provides a method of differentiating stem cells into corneal endothelial cells (CEC), the method including culturing the stem cells in a culture medium containing a Rho-associated protein kinase (ROCK) inhibitor to differentiate the stem cells into corneal endothelial cells.

In the present specification, the term "stem cell" may refer to a totipotent cell capable of differentiating into all types of cells, or a pluripotent cell capable of differentiating into multiple types of cells, and a stem cell is an undifferentiated cell that can differentiate into the cells of a specific tissue.

In an embodiment, the stem cells may be embryonic stem cells (ESC), adult stem cells, or induced pluripotent stem cells (iPSC).

The embryonic stem cells may be cells cultured in vitro after extraction from the inner cell mass of a pre-implantation blastocyst, and the adult stem cells may be undifferentiated cells found in minute quantities in various tissues of the body, where they can replace dead cells or damaged tissue. The induced pluripotent stem cells (iPSC) may be cells induced to pluripotency, like embryonic stem cells, by reverting differentiated somatic cells to a pre-differentiation cell stage through introduction of cell differentiation-related genes.

In the present specification, the term "corneal endothelial cell (CEC)" may refer to a cell constituting the corneal endothelium.

In the present specification, the term "differentiation" may refer to a phenomenon in which cells become specialized in structure or function during growth through cell division and proliferation, that is, a process in which the morphology or function of cells or tissues of a living organism changes to perform their given roles. In addition, in the present specification, the term "differentiation potential" may refer to the ability to differentiate, and a "cell having differentiation potential" may refer to a cell that has the ability to differentiate into the tissues or cells constituting a living organism.

In the present specification, the term "inducing differentiation of stem cells into corneal endothelial cells" may include not only cases in which complete differentiation into corneal endothelial cells is induced from stem cells, including iPSCs, but also cases in which differentiation is induced into cells expressing corneal endothelial cell-specific markers.

In an embodiment, the culture medium may induce differentiation of stem cells into corneal endothelial cells by downregulating characteristics of the cells as pluripotent stem cells and upregulating characteristics specific to corneal endothelial cells during the induction process of differentiation of the stem cells into corneal endothelial cells. For example, the culture medium may decrease the expression of pluripotent stem cell-specific markers or increase the expression of corneal endothelial cell-specific markers.

The pluripotent stem cell-specific marker may include one or more selected from the group consisting of OCT4 (octamer-binding transcription factor 4), SOX2 (SRY-Box Transcription Factor 2), and NANOG, but is not limited thereto.

The corneal endothelial cell-specific marker may include one or more selected from the group consisting of ZO-1 (Zonula occludens-1), NCAM (neuronal cell adhesion molecule), SLC4A11 (Solute Carrier Family 4 Member 11), N-cadherin, COL8A1 (Collagen Type VIII Alpha 1 Chain), AQP1 (Aquaporin 1), and ATP1A1 (ATPase Na⁺/K⁺ Transporting Subunit Alpha 1), but is not limited thereto.

In an embodiment, the method of differentiating stem cells into corneal endothelial cells may include culturing the stem cells in a culture vessel having a modified extracellular matrix (ECM), in the presence of a composition containing a Rho-associated protein kinase (ROCK) inhibitor.

In an embodiment, the method of differentiating stem cells into corneal endothelial cells may further include culturing the stem cells in a culture vessel modified with an extracellular matrix (ECM). For example, the culturing of the stem cells may be carried out for 3 to 8 days, but is not limited thereto.

In an embodiment, the extracellular matrix (ECM) may include one or more selected from the group consisting of vitronectin, collagen, fibronectin, laminin, gelatin, matrigel, hyaluronic acid, polylysine, and heparan sulfate proteoglycan, but is not limited thereto. In an embodiment, the method of differentiating may include culturing in a culture vessel containing the culture medium for 1 to 20 days. For example, the differentiating of the stem cells into corneal endothelial cells may include culturing in a culture vessel containing the culture medium for 1 to 20 days, for example, 1 to 19 days, 1 to 18 days, 1 to 17 days, 1 to 16 days, 1 to 15 days, 1 to 14 days, 1 to 13 days, 1 to 12 days, 1 to 11 days, 1 to 10 days, 1 to 9 days, 1 to 8 days, 1 to 7 days, 1 to 6 days, 1 to 5 days, 2 to 20 days, 2 to 19 days, 2 to 18 days, 2 to 17 days, 2 to 16 days, 2 to 15 days, 2 to 14 days, 2 to 13 days, 2 to 12 days, 2 to 11 days, 2 to 10 days, 2 to 9 days, 2 to 8 days, 2 to 7 days, 2 to 6 days, 2 to 5 days, 3 to 20 days, 3 to 19 days, 3 to 18 days, 3 to 17 days, 3 to 16 days, 3 to 15 days, 3 to 14 days, 3 to 13 days, 3 to 12 days, 3 to 11 days, 3 to 10 days, 3 to 9 days, 3 to 8 days, 3 to 7 days, 3 to 6 days, 3 to 5 days, 4 to 20 days, 4 to 19 days, 4 to 18 days, 4 to 17 days, 4 to 16 days, 4 to 15 days, 4 to 14 days, 4 to 13 days, 4 to 12 days, 4 to 11 days, 4 to 10 days, 4 to 9 days, 4 to 8 days, 4 to 7 days, 4 to 6 days, 4 to 5 days, 5 to 20 days, 5 to 19 days, 5 to 18 days, 5 to 17 days, 5 to 16 days, 5 to 15 days, 5 to 14 days, 5 to 13 days, 5 to 12 days, 5 to 11 days, 5 to 10 days, 5 to 9 days, 5 to 8 days, 5 to 7 days, 6 to 20 days, 6 to 19 days, 6 to 18 days, 6 to 17 days, 6 to 16 days, 6 to 15 days, 6 to 14 days, 6 to 13 days, 6 to 12 days, 6 to 11 days, 6 to 10 days, 6 to 9 days, 6 to 8 days, 7 to 20 days, 7 to 19 days, 7 to 18 days, 7 to 17 days, 7 to 16 days, 7 to 15 days, 7 to 14 days, 7 to 13 days, 7 to 12 days, 7 to 11 days, 7 to 10 days, 7 to 9 days, 8 to 20 days, 8 to 19 days, 8 to 18 days, 8 to 17 days, 8 to 16 days, 8 to 15 days, 8 to 14 days, 8 to 13 days, 8 to 12 days, 8 to 11 days, 8 to 10 days, 9 to 20 days, 9 to 19 days, 9 to 18 days, 9 to 17 days, 9 to 16 days, 9 to 15 days, 9 to 14 days, 9 to 13 days, 9 to 12 days, 9 to 11 days, 10 to 20 days, 10 to 19 days, 10 to 18 days, 10 to 17 days, 10 to 16 days, 10 to 15 days, 10 to 14 days, 10 to 13 days, 10 to 12 days, 11 to 20 days, 11 to 19 days, 11 to 18 days, 11 to 17 days, 11 to 16 days, 11 to 15 days, 11 to 14 days, 11 to 13 days, 12 to 20 days, 12 to 19 days, 12 to 18 days, 12 to 17 days, 12 to 16 days, 12 to 15 days, 12 to 14 days, 13 to 20 days, 13 to 19 days, 13 to 18 days, 13 to 17 days, 13 to 16 days, 14 to 20 days, 14 to 19 days, 14 to 18 days, 14 to 17 days, 14 to 16 days, 15 to 20 days, 15 to 19 days, 15 to 18 days, or 15 to 17 days.

The culturing may be performed under a temperature condition of 30 °C to 40 °C, for example, 35 °C to 38 °C, and specifically, under a temperature condition of 37 °C.

In an embodiment, the method of differentiating may not include a step of differentiating the stem cells into neural crest cells (NCCs).

In the present specification, the term "not including a step of differentiating into neural crest cells (NCCs)" may refer to a case in which, during the process of differentiating stem cells into corneal endothelial cells, no NCC morphology is observed and no NCC-specific genes are expressed.

In the present specification, the term "neural crest cell (NCC)" may refer to a cell generated at the boundary between the neural tube and the epidermal ectoderm during central nervous system formation in embryonic development, which migrates to various regions in the body through epithelial-mesenchymal transition (EMT).

In an embodiment, the process of differentiating may refer to directly differentiating an induced pluripotent stem cell (iPSC) into a corneal endothelial cell (CEC).

In the present specification, the term "direct differentiation" may refer to a process in which a stem cell directly develops or specializes into a specific type of cell, that is, a process that proceeds directly to the target cell type without passing through an intermediate cell type. For example, the term may refer to differentiating a stem cell directly into a corneal endothelial cell (CEC) without a neural crest cell (NCC) stage.

In the present specification, the term "Rho-associated protein kinase (ROCK) inhibitor" may refer to a substance that inhibits Rho-associated protein kinase, and a ROCK inhibitor may be used interchangeably with a ROCK suppressor.

In an embodiment, the ROCK inhibitor may include one or more selected from the group consisting of fasudil, N-(4-pyridyl)-4β-[(R)-1-aminoethyl]cyclohexane-1α-carboxamide (Y-27632), (2S)-2-methyl-1-[(4-methyl-5-isoquinolinyl)sulfonyl]hexahydro-1H-1,4-diazepine (H-1152), 4-[(1R)-1-aminoethyl]-N-pyridin-4-ylbenzamide hydrochloride (Wf-536), (1R,4r)-4-((R)-1-aminoethyl)-N-(1H-pyrrolo[2,3-b]pyridin-4-yl)cyclohexane-1-carboxamide (Y-30141), N-(3-{[2-(4-amino-1,2,5-oxadiazol-3-yl)-1-ethyl-1H-imidazo[4,5-c]pyridin-6-yl]oxy}phenyl)-4-{[2-(4-morpholinyl)ethyl]oxy}benzamide (GSK269962A), and N-(6-fluoro-1H-indazol-5-yl)-6-methyl-2-oxo-4-[4-(trifluoromethyl)phenyl]-3,4-dihydro-1H-pyridine-5-carboxamide (GSK429286A).

In an embodiment, the medium may further include a component suitable for inducing differentiation of the stem cells into corneal endothelial cells (CECs). For example, the "component suitable for inducing differentiation of the stem cells into corneal endothelial cells (CECs)" may be one or more selected from the group consisting of a TGF-beta inhibitor, insulin-transferrin-selenium (ITS), ascorbic acid, calcium chloride (CaCl₂), and epidermal growth factor (EGF), but is not limited thereto.

In an embodiment, the culture medium may further include a TGF-beta inhibitor or insulin-transferrin-selenium (ITS).

In an embodiment, the TGF-beta inhibitor may include one or more selected from the group consisting of 4-[4-(2H-1,3-benzodioxol-5-yl)-5-(pyridin-2-yl)-1H-imidazol-2-yl]benzamide (SB431542), 4-(6-(4-(piperazin-1-yl)phenyl)pyrazolo[1,5-a]pyrimidin-3-yl)quinoline (LDN-193189), 4-(2-(6-methylpyridin-2-yl)-5,6-dihydro-4H-pyrrolo[1,2-b]pyrazol-3-yl)quinoline-6-carboxamide (LY-2157299), 4-(2-((4-(2-(pyridin-2-yl)-5,6-dihydro-4H-pyrrolo[1,2-b]pyrazol-3-yl)quinolin-7-yl)oxy)ethyl)morpholine (LY-2109761), 6-(2-(tert-butyl)-4-(6-methylpyridin-2-yl)-1H-imidazol-5-yl)quinoxaline (SB-525334), 2-(3-(6-methylpyridin-2-yl)-1H-pyrazol-4-yl)-1,5-naphthyridine (E-616452), 4-(3-(pyridin-2-yl)-1H-pyrazol-4-yl)quinoline (LY-364947), and 2-(4-((4-((1-cyclopropyl-3-(tetrahydro-2H-pyran-4-yl)-1H-pyrazol-4-yl)oxy)pyridin-2-yl)amino)pyridin-2-yl)propan-2-ol (LY-3200882). For example, the culture medium may be a medium including Human Endothelial-SFM, including SB431542, fasudil, H-1152, and insulin-transferrin-selenium (ITS) as medium components, but is not limited thereto.

In an embodiment, the culture medium may further include ascorbic acid, calcium chloride (CaCl₂), or epidermal growth factor (EGF). For example, the culture medium may further include calcium chloride, 2-phosphate ascorbic acid, EGF, or a combination thereof, but is not limited thereto.

In an embodiment, the culture medium may not comprise fetal bovine serum (FBS).

In the present specification, the terms "substantially does not comprise" or "does not comprise" may refer to a case in which fetal bovine serum is included in an amount that does not affect the physical properties, activity, or pharmacological activity of the composition, or is not included at all. For example, the term may include a case in which fetal bovine serum is comprised in an amount of 0.02 % by weight or less, 0.01 % by weight or less, 0.005 % by weight or less, or 0.001 % by weight or less, based on the total weight of the composition, or is not comprised at all.

Another aspect provides a culture medium composition for inducing direct differentiation of stem cells into corneal endothelial cells, the culture medium composition containing a Rho-associated protein kinase (ROCK) inhibitor.

In the present specification, the term "direct differentiation" may refer to a process in which a stem cell directly develops or specializes into a specific type of cell without passing through an intermediate cell type, that is, a process that proceeds directly to the target cell type. For example, the term may refer to differentiating directly from a stem cell into a corneal endothelial cell (CEC) without passing through a neural crest cell (NCC).

In an embodiment, the ROCK inhibitor may include one or more selected from the group consisting of fasudil, N-(4-pyridyl)-4β-[(R)-1-aminoethyl]cyclohexane-1α-carboxamide (Y-27632), (2S)-2-methyl-1-[(4-methyl-5-isoquinolinyl)sulfonyl]hexahydro-1H-1,4-diazepine (H-1152), 4-[(1R)-1-aminoethyl]-N-pyridin-4-ylbenzamide hydrochloride (Wf-536), (1R,4r)-4-((R)-1-aminoethyl)-N-(1H-pyrrolo[2,3-b]pyridin-4-yl)cyclohexane-1-carboxamide (Y-30141), N-(3-{[2-(4-amino-1,2,5-oxadiazol-3-yl)-1-ethyl-1H-imidazo[4,5-c]pyridin-6-yl]oxy}phenyl)-4-{[2-(4-morpholinyl)ethyl]oxy}benzamide (GSK269962A), and N-(6-fluoro-1H-indazol-5-yl)-6-methyl-2-oxo-4-[4-(trifluoromethyl)phenyl]-3,4-dihydro-1H-pyridine-5-carboxamide (GSK429286A), but is not limited thereto.

In an embodiment, the culture medium composition may further include a TGF-beta inhibitor or insulin-transferrin-selenium (ITS).

In an embodiment, the TGF-beta inhibitor may include one or more selected from the group consisting of 4-[4-(2H-1,3-benzodioxol-5-yl)-5-(pyridin-2-yl)-1H-imidazol-2-yl]benzamide (SB431542), 4-(6-(4-(piperazin-1-yl)phenyl)pyrazolo[1,5-a]pyrimidin-3-yl)quinoline (LDN-193189), 4-(2-(6-methylpyridin-2-yl)-5,6-dihydro-4H-pyrrolo[1,2-b]pyrazol-3-yl)quinoline-6-carboxamide (LY-2157299), 4-(2-((4-(2-(pyridin-2-yl)-5,6-dihydro-4H-pyrrolo[1,2-b]pyrazol-3-yl)quinolin-7-yl)oxy)ethyl)morpholine (LY-2109761), 6-(2-(tert-butyl)-4-(6-methylpyridin-2-yl)-1H-imidazol-5-yl)quinoxaline (SB-525334), 2-(3-(6-methylpyridin-2-yl)-1H-pyrazol-4-yl)-1,5-naphthyridine (E-616452), 4-(3-(pyridin-2-yl)-1H-pyrazol-4-yl)quinoline (LY-364947), and 2-(4-((4-((1-cyclopropyl-3-(tetrahydro-2H-pyran-4-yl)-1H-pyrazol-4-yl)oxy)pyridin-2-yl)amino)pyridin-2-yl)propan-2-ol (LY-3200882), but is not limited thereto.

In an embodiment, the medium composition may further include ascorbic acid, calcium chloride (CaCl₂), or epidermal growth factor (EGF), but is not limited thereto.

The medium composition may additionally include, without limitation, components that are conventionally used for culturing and/or differentiating stem cells. For example, the medium may include various nutrients required for maintaining and proliferating cells or components required for inducing differentiation. Specifically, as the nutrient source, the medium may include carbon sources such as glycerol, glucose, fructose, sucrose, lactose, honey, starch, and dextrin; hydrocarbons such as fatty acids, oils and fats, lecithin, and alcohols; nitrogen sources such as ammonium sulfate, ammonium nitrate, ammonium chloride, urea, and sodium nitrate; inorganic salts such as sodium chloride, potassium salts, phosphates, magnesium salts, calcium salts, iron salts, and manganese salts; monopotassium phosphate, dipotassium phosphate, magnesium sulfate, sodium chloride, ferrous sulfate, sodium molybdate, sodium tungstate, and manganese sulfate; and various vitamins and amino acids, but is not limited thereto.

Another aspect provides a kit for inducing direct differentiation of stem cells into corneal endothelial cells, including: a composition containing a Rho-associated protein kinase (ROCK) inhibitor; and a culture vessel modified with an extracellular matrix (ECM).

In the present specification, the term "extracellular matrix (ECM)" may refer to a complex assembly of biomacromolecules that fills spaces within tissues or outside cells, is synthesized by cells, and consists of molecules secreted and accumulated extracellularly. The term "analog of extracellular matrix" may refer to a mixture having a composition similar to an extracellular matrix consisting of a three-dimensional network of extracellular macromolecules, that is an artificial or natural protein mixture that performs the same or similar biological function as a natural extracellular matrix.

In an embodiment, the extracellular matrix may include one or more selected from the group consisting of vitronectin, collagen, fibronectin, laminin, gelatin, matrigel, hyaluronic acid, polylysine, and heparan sulfate proteoglycan.

In the present specification, the term "culture vessel" may mean a cell culture plate or the like, which is typically used for cell culture or biochemical experiments, but is not particularly limited thereto, and may, for example, include dishes, flasks, and multi-well plates.

In the present specification, the term "modified (coating)" may refer to forming a new layer of a certain thickness by forming a film of a specific material on a target surface. The target surface and the modifying material may be coated via various chemical bonds such as ionic bonds, covalent bonds, and hydrogen bonds. When the surface of a culture vessel is modified with an extracellular matrix or the like, the extracellular matrix may completely surround the surface of the culture vessel to form a closed layer or may form a partially closed layer.

The ROCK inhibitor, composition, extracellular matrix, culture vessel, stem cell, and corneal endothelial cell are as described above.

### Advantageous Effects of Invention

According to the method of one aspect, using the differentiation composition of the present disclosure can reduce the time and cost of differentiating stem cells into corneal endothelial cells, thereby increasing differentiation efficiency, and the differentiated corneal endothelial cells exhibit the specific characteristics of corneal endothelial cells and thus can be widely used as an alternative cell source for corneal endothelial cell transplantation.

### Description of Drawings

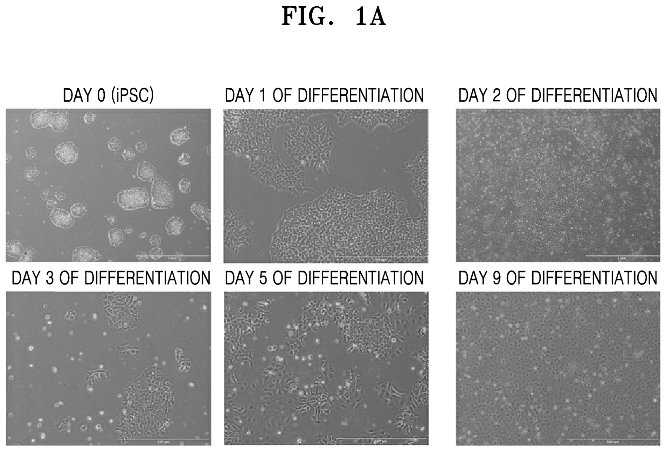

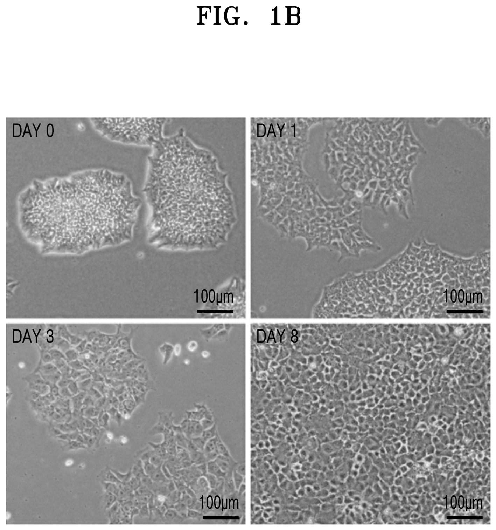

FIGS. 1A and 1B are images photographed under a microscope to observe morphological changes during the differentiation process from hiPSC to CEC according to an aspect. The images show hiPSCs, and cells at day 1, day 2, day 3, day 5, and day 9 of differentiation into CEC induced from the hiPSCs.
FIG. 1A is an image photographed under a microscope at 40X magnification of hiPSCs, and cells at day 1, day 2, day 3, day 5, and day 9 of differentiation into CEC induced from the hiPSCs, during the differentiation process from hiPSC to CEC according to an aspect. FIG. 1B is an image photographed under a microscope at 100X magnification of hiPSCs, and cells at day 1, day 3, and day 8 of differentiation into CEC induced from the hiPSCs, during the differentiation process from hiPSC to CEC according to an aspect.
FIG. 2 shows the results of measuring the expression of characteristic markers of corneal endothelial cells, namely ZO-1 (Zonula occludens-1), CD166, and ATP1A1 (ATPase Na+/K+ Transporting Subunit Alpha 1), in corneal endothelial cells directly differentiated from iPSCs.
FIG. 3 is a graph showing the expression levels of the corneal endothelial cell-specific gene (CDH2) and iPSC-specific genes (LIN28A, OCT4) at 12 days after direct differentiation of iPSCs into CECs in a culture medium according to an aspect.
FIG. 4 is an image obtained by confirming, through immunocytochemical staining, whether ZO-1 was expressed in CECs directly differentiated from iPSCs in the culture media of Example 3-1 and Examples 3-3 to 3-7.
FIG. 5 is a photograph obtained by confirming, through Western blotting, the protein expression levels of each of the corneal endothelial cell-specific markers (ZO-1, N-cadherin, ATP1A1, and SLC4A11) in CECs directly differentiated from iPSCs and cultured for 12 days in the culture media of Examples 3-1 and 3-3 according to an aspect.
FIGS. 6A to 6C are graphs showing the results of functional annotation analysis performed through single cell sequencing.

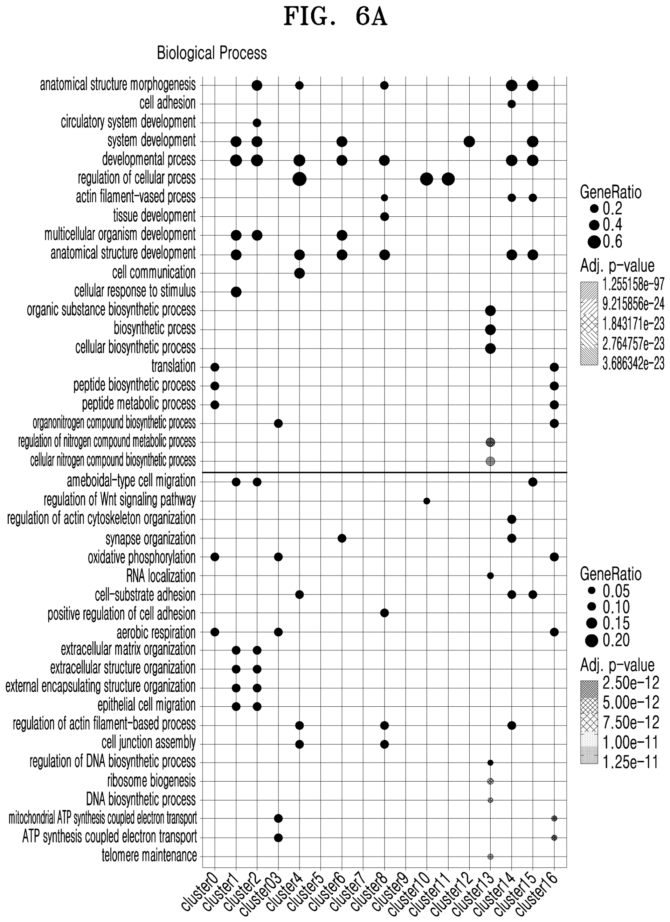

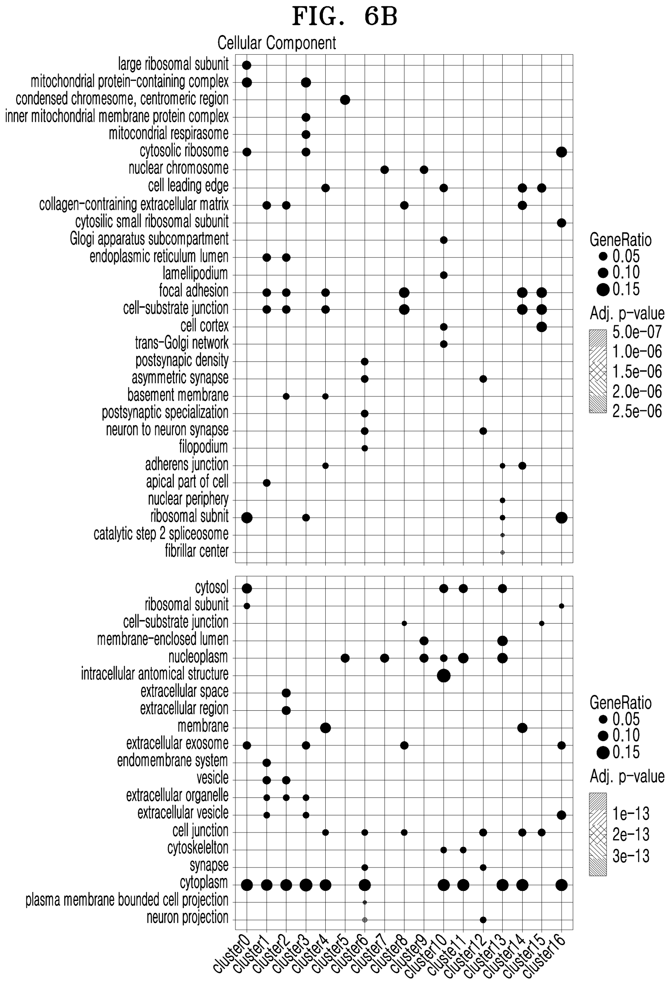

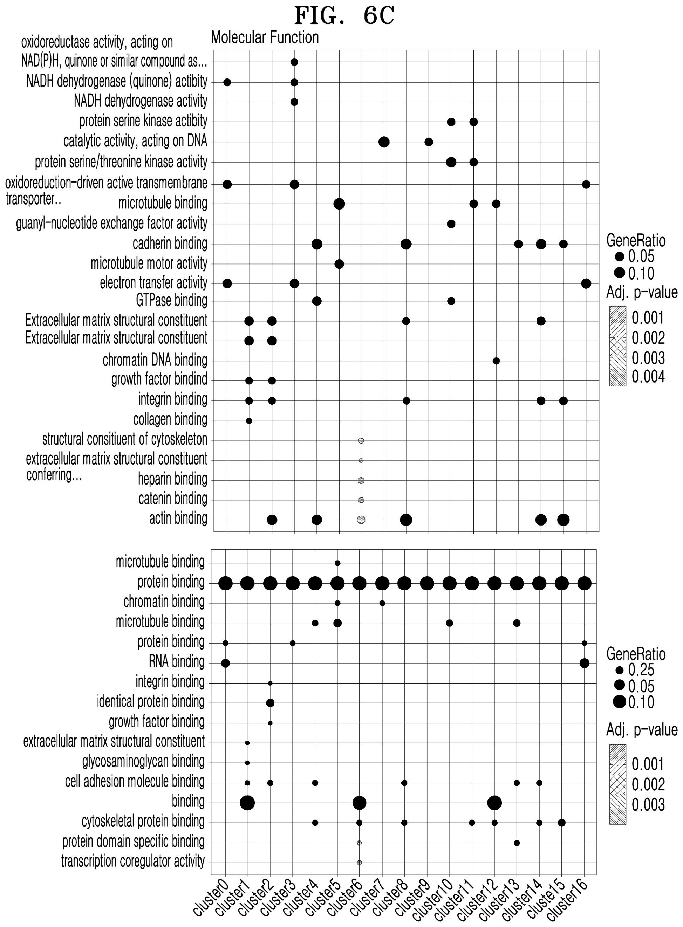

FIG. 6A shows results for biological processes, FIG. 6B shows results for cellular components, and FIG. 6C is a graph showing results for molecular functions.

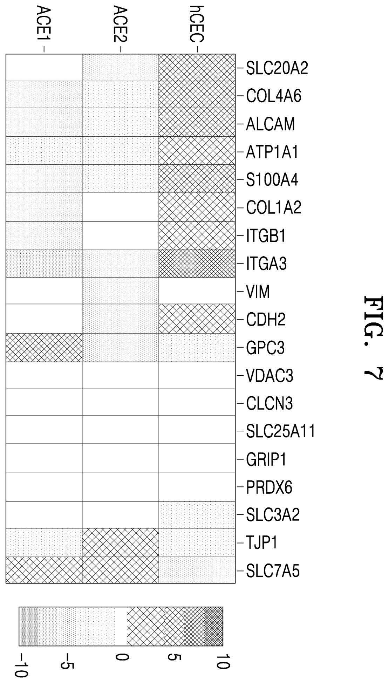

FIG. 7 is a heat map comparing the expression patterns of various corneal endothelial cell-specific gene markers among the hCEC (human-derived corneal endothelial cell) group, the ACE1 (CEC differentiated from iPSCs via NCC) group, and the ACE2 (CEC directly differentiated from iPSCs) group.

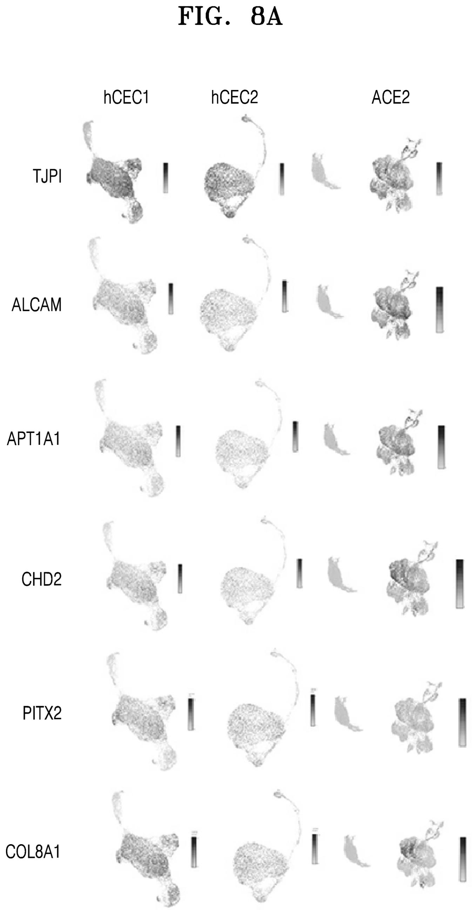

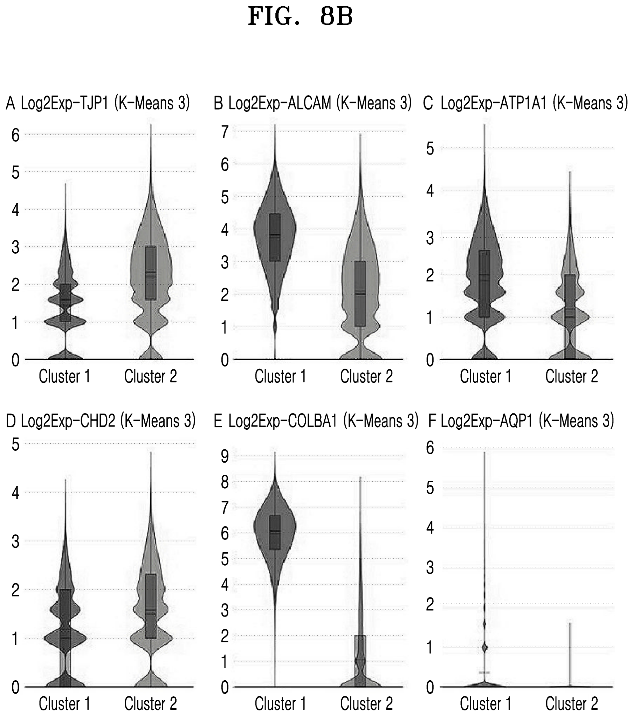

FIGS. 8A and 8B are graphs analyzing the expression patterns of corneal endothelial cell-specific gene markers (TJP1,ALCAM, ATP1A1, CDH2, COL8A1, and AQP1) in the hCEC (human-derived corneal endothelial cell) group and the ACE2 (corneal endothelial cells directly differentiated from induced pluripotent stem cells) group.
FIG. 8A shows the results of comparing, at the single cell level through uniform manifold approximation and projection (UMAP), the expression of corneal endothelial cell-specific gene markers (TJP1, ALCAM, ATP1A1, CDH2, COL8A1, and AQP1) in the hCEC (human-derived corneal endothelial cell) group and the ACE2 (corneal endothelial cells directly differentiated from induced pluripotent stem cells) group. FIG. 8B is a graph analyzing the expression patterns of corneal endothelial cell-specific gene markers (TJP1, ALCAM, ATP1A1, CDH2, COL8A1, and AQP1) in the hCEC group (Cluster 1, blue) and the ACE2 group (Cluster 2, orange).

### Mode for Invention

Hereinafter, the present disclosure will be described in greater detail by way of Examples. However, these Examples are provided for illustrative purposes only and are not intended to limit the scope of the present disclosure.

### Comparative Example 1. Method of differentiating stem cells into corneal endothelial cells via neural crest cells (NCCs)

Human induced pluripotent stem cells (hiPSCs) were obtained from YiPSCELL Inc. The hiPSCs were cultured on vitronectin-coated plates in E8 medium under feeder-free and serum-free conditions.

For differentiation of the hiPSCs into neural crest cells (NCCs), the hiPSCs were plated at a low density on the surface of a vitronectin-coated plate. After the undifferentiated hiPSCs reached about 70 to 80 % confluency, the first medium was added to the plate to induce differentiation into neural crest cells. The first medium used was as follows: more specifically, the culture medium was replaced with neural crest cell induction E8 medium (Life Technologies) supplemented with 10 µM retinoic acid (Sigma-Aldrich) and 5 µM CHIR99021 (BioGems Korea), and the cells were cultured for one day. The culture medium was then replaced with E8 medium supplemented with 5 µM retinoic acid, 10 µM SB431542 (Selleckchem), 5 µM CHIR99021, and 500 nM LDN193189 (Selleckchem), and cultured for one day. The medium was then replaced with E8 medium supplemented with 5 µM retinoic acid, 10 µM SB431542, 5 µM CHIR99021, and 100 nM BGJ398 (Selleckchem), and cultured for one day. Thereafter, the culture medium was replaced daily with neurobasal medium with neural induction supplements (Life Technologies) for 10 to 14 days to induce differentiation into neural crest cells.

For differentiation of the induced NCCs into corneal endothelial cells (CECs), the iPSC-derived NCCs were dissociated using TrypLE Express (Life Technologies). The cells were seeded at a low confluency (20 to 30 %) on 35 mm vitronectin-coated plates. The cells were then cultured for 14 days in a second medium containing human endothelial-SFM supplemented with 10 ng/ml hEGF (Life Technologies), 1 % insulin-transferrin-selenium (Life Technologies), 0.2 mg/mL CaCl₂ (Sigma-Aldrich), 0.02 mg/mL 2-phosphate ascorbic acid, 1 µM SB431542 (Selleckchem), 2.5 µM ROCK inhibitor H-1152 (Tocris, Abington, UK), and 10 µM Fasudil (Stemcell Technologies) to induce differentiation into corneal endothelial cells. When the cells differentiated into corneal endothelial cells reached about 80 to 90 % confluency, they were subcultured.

### Example 1. Method of differentiating stem cells directly into corneal endothelial cells

Human induced pluripotent stem cells (hiPSCs) were obtained from YiPSCELL Inc. To induce direct differentiation of the hiPSCs into corneal endothelial cells without undergoing differentiation into neural crest cells (NCCs), the hiPSCs were plated at a low density on the surface of a vitronectin-coated plate. After the undifferentiated hiPSCs reached a confluency of about 70 to 80 %, culture medium suitable for direct differentiation into corneal endothelial cells was added to the plate to induce differentiation into corneal endothelial cells.

Specifically, the iPSCs were first cultured for 4 to 5 days using E8 medium (Life Technologies). Thereafter, the cells were seeded at a low confluency (20 to 30 %) onto a 35 mm plate coated with vitronectin. Subsequently, the cells were induced to differentiate into corneal endothelial cells for 10 to 14 days at 37 °C, while replacing the medium daily, in a medium supplemented with 10 ng/mL human epidermal growth factor (hEGF) (Life Technologies), 1 % insulin-transferrin-selenium (Life Technologies), 0.2 mg/mL CaCl₂ (Sigma-Aldrich), 0.02 mg/mL 2-phosphate ascorbic acid, 1 µM SB431542 (Selleckchem), 2.5 µM ROCK inhibitor H-1152 (Tocris, Abington, UK), and 10 µM Fasudil (Stemcell Technologies).

### Example 2. Verification of characteristics of corneal endothelial cells differentiated from iPSCs

### 2-1. Verification of morphological development of corneal endothelial cells

To verify whether the phenotype of corneal endothelial cells (CECs) occurs during the direct differentiation from hiPSCs to CECs according to the method of Example 1, cells at day 1, day 2, day 3, day 5, and day 9 of direct differentiation from hiPSCs to CECs were observed under a microscope for their morphology, and the results are shown in FIGS. 1A and 1B.

FIGS. 1A and 1B are images photographed under a microscope to observe morphological changes during the differentiation process from hiPSC to CEC according to an aspect. The images show hiPSCs, and cells at day 1, day 2, day 3, day 5, and day 9 of differentiation into CEC induced from the hiPSCs.

FIG. 1A is an image photographed under a microscope at 40X magnification of hiPSCs, and cells at day 1, day 2, day 3, day 5, and day 9 of differentiation into CEC induced from the hiPSCs, during the differentiation process from hiPSC to CEC according to an aspect. FIG. 1B is an image photographed under a microscope at 100X magnification of hiPSCs, and cells at day 1, day 3, and day 8 of differentiation into CEC induced from the hiPSCs, during the differentiation process from hiPSC to CEC according to an aspect.

As shown in FIGS. 1A and 1B, from day 7 to day 9 of direct differentiation from hiPSCs to CECs according to the method of Example 1, it was confirmed that the cells exhibited hexagonal shapes and tight intercellular junctions, which are representative characteristics of corneal endothelial cells.

### 2-2. Verification of expression of corneal endothelial cell-specific markers via immunocytochemical staining

The expression of corneal endothelial cell-specific markers in CECs directly differentiated from hiPSCs according to the method of Example 1 was verified.

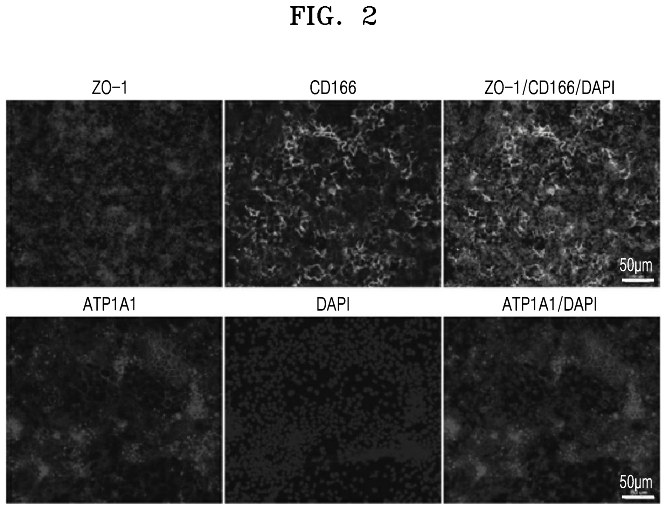

Specifically, the expression of ZO-1 (Zonula occludens-1), CD166, and ATP1A1 (ATPase Na+/K+ Transporting Subunit Alpha 1), which are corneal endothelial cell-specific markers, was measured via immunocytochemical staining. The CECs were fixed with 4 % paraformaldehyde for 30 minutes, washed with PBS buffer, permeabilized with 0.5 % Triton X-100, and blocked in PBST containing 1 % bovine serum albumin (Sigma-Aldrich). The samples were incubated overnight at 4 °C with anti-human ZO-1 antibody (1:250; Life Technologies) as the primary antibody, followed by incubation with goat anti-mouse IgG Alexa Fluor 488-conjugated and donkey anti-rabbit IgG Alexa Fluor 647-conjugated secondary antibodies (1:500; Abcam, Cambridge, UK). The nuclei were counterstained for 10 minutes with 4',6-diamidino-2-phenylindole (DAPI; Sigma-Aldrich), and the fluorescence signals were detected using a laser scanning confocal microscope (Olympus, Tokyo, Japan) or a fluorescence microscope (EVOS, Life Technologies). The results are shown in FIG. 2.

FIG. 2 shows the results of measuring the expression of ZO-1 (Zonula occludens-1), CD166, and ATP1A1 (ATPase Na+/K+ Transporting Subunit Alpha 1), which are characteristic markers of corneal endothelial cells, in corneal endothelial cells directly differentiated from iPSCs.

As shown in FIG. 2, the CECs directly differentiated from iPSCs according to the method of Example 1 showed clear expression of the corneal endothelial cell-specific markers ZO-1 (Zonula occludens-1), CD166, and ATP1A1 (ATPase Na+/K+ Transporting Subunit Alpha 1).

### Example 3. Composition of culture medium used for direct differentiation of human induced pluripotent stem cells (hiPSCs) into corneal endothelial cells

To identify the essential components among the constituents of the culture medium used for direct differentiation of human induced pluripotent stem cells (hiPSCs) into corneal endothelial cells (CECs) by the method of Example 1, direct differentiation from hiPSCs into CECs was induced using various combinations of culture media.

The composition of the culture medium used to induce direct differentiation from hiPSCs into CECs is shown in Table 1 below.

**[Table 1]**

| Constituents | Example 3-1 | Example 3-2 | Example 3-3 | Example 3-4 | Example 3-5 | Example 3-6 | Example 3-7 |
|---|---|---|---|---|---|---|---|
| SB431542 | ○ | ○ | ○ | ○ | ○ | - | ○ |
| Fasudil | ○ | ○ | ○ | ○ | ○ | ○ | - |
| H-1152 | ○ | ○ | ○ | ○ | - | ○ | ○ |
| ITS | ○ | ○ | ○ | - | ○ | ○ | ○ |
| AA(2-phosphate ascorbic acid) | ○ | ○ | - | - | - | - | - |
| CaCl₂ | ○ | ○ | - | - | - | - | - |
| EGF | ○ | - | - | - | - | - | - |

### Example 4. Verification of characteristics according to culture medium composition for direct differentiation of human induced pluripotent stem cells (hiPSCs) into corneal endothelial cells

### 4-1. Verification of stability of corneal endothelial cells differentiated from iPSCs

To confirm the stability of CECs directly differentiated from iPSCs according to the culture medium constituents for direct differentiation of iPSCs into CECs, the expression levels of a CEC-specific gene (CDH2) and iPSC-specific genes (LIN28A, OCT4) were measured by RT-PCR in cells differentiated in the culture media of Examples 3-1 to 3-3.

Specifically, iPSCs were cultured in the culture media of Examples 3-1 to 3-3, and CECs directly differentiated from the iPSCs were obtained after 12 days. Total RNA of the CECs directly differentiated from the iPSCs was extracted using TRIzol reagent (Invitrogen), and complementary DNA was synthesized using a kit (Superscript III; Invitrogen). Quantitative RT-PCR (qRT-PCR) was performed on a Step One ABI Real-Time PCR System (Applied Biosystems) using Power SYBR Green PCR Master Mix (Applied Biosystems, CA, USA).

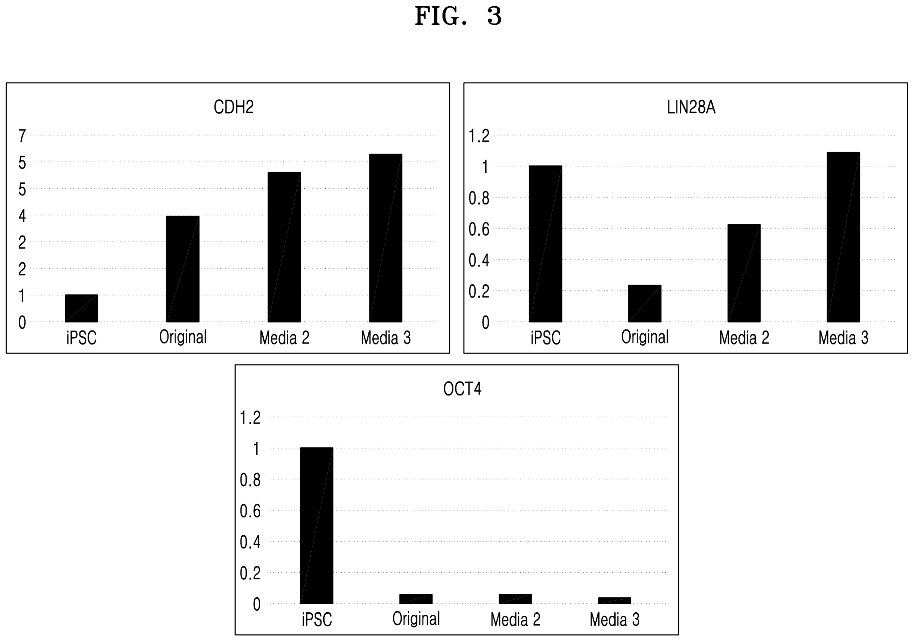

The expression levels of the CEC-specific gene (CDH2) and the iPSC-specific genes (LIN28A, OCT4) in the CECs directly differentiated from the iPSCs cultured in the media of Examples 3-1 to 3-3 were measured, and the results are shown in FIG. 3.

FIG. 3 is a graph showing the expression levels of the CEC-specific gene (CDH2) and the iPSC-specific genes (LIN28A, OCT4) measured at 12 days after direct differentiation from iPSCs into CECs in the culture media according to an aspect.

As shown in FIG. 3, when iPSCs were directly differentiated into CECs using the culture media of Examples 3-1 to 3-3, the expression of the CEC-specific gene (CDH2) was further increased in Examples 3-2 and 3-3, compared to Example 3-1. In addition, when comparing the expression levels of the iPSC-specific genes (LIN28A, OCT4), Example 3-1 showed a reduction in expression compared to Examples 3-2 and 3-3.

This indicates that the culture medium compositions of Examples 3-2 and 3-3 have an effect of promoting direct differentiation from iPSCs into CECs, and that the culture medium composition of Example 3-1 has an effect of promoting corneal endothelial cell differentiation while simultaneously reducing iPSC characteristics.

### 4-2. Comparison of the efficiency of differentiation of iPSCs into corneal endothelial cells

The direct differentiation efficiency from iPSCs into CECs was evaluated using the culture media of Examples 3-1 to 3-7, in which certain constituents of the culture medium of Example 3-1 for direct differentiation of iPSCs into CECs were omitted.

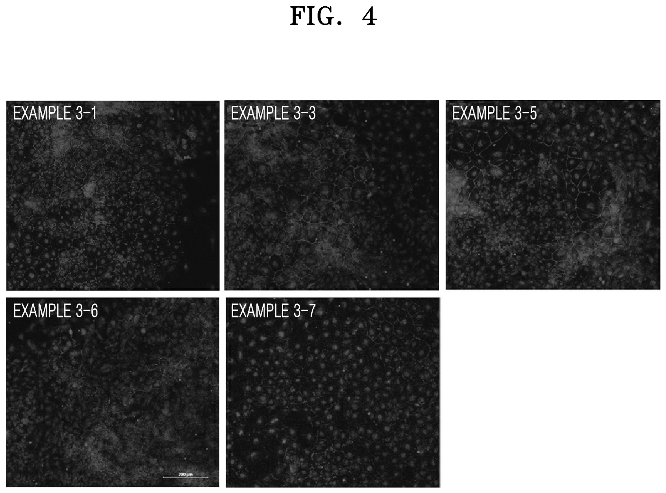

Specifically, CECs were directly differentiated from iPSCs by the same method as in Example 1, except that the culture media of Examples 3-1 to 3-7 were used, and the expression level of the CEC-specific marker (ZO-1) was examined in the resulting CECs. To compare the effect on differentiation efficiency using ZO-1 marker staining of iPSC-derived CECs, CECs were fixed in 4 % paraformaldehyde for 30 minutes, washed with PBS buffer, permeabilized with 0.5 % Triton X-100, and blocked in PBST containing 1% bovine serum albumin (Sigma-Aldrich). Samples were incubated overnight at 4 °C with a primary human anti-ZO-1 antibody (1:250; Life Technologies), followed by incubation with secondary antibodies, goat anti-mouse IgG Alexa Fluor 488-conjugated and donkey anti-rabbit IgG Alexa Fluor 647-conjugated antibodies (1:500; Abcam, Cambridge, UK). Nuclei were counterstained for 10 minutes with 4',6-diamidino-2-phenylindole (DAPI; Sigma-Aldrich), and fluorescence signals were detected using a laser scanning confocal microscope (Olympus, Tokyo, Japan) or a fluorescence microscope (EVOS, Life Technologies). The results are shown in FIG. 4.

FIG. 4 is an image from immunocytochemical staining, confirming the expression of ZO-1 in CECs directly differentiated from iPSCs in the culture media of Example 3-1 and Examples 3-3 to 3-7.

As shown in FIG. 4, in the culture media of Example 3-1 and Examples 3-3 to 3-7 according to an aspect, most of the differentiated cells expressed ZO-1, but particularly, the groups of CECs directly differentiated from iPSCs in the culture media of Example 3-1 and Example 3-3 showed the best cell differentiation and growth. On the other hand, cells differentiated in the culture medium of Example 3-4 did not grow well.

This indicates that in culture media lacking ITS, direct differentiation from iPSCs into CECs does not occur efficiently, and that Fasudil, SB431542, and H-1152 are important for direct differentiation from iPSCs into CECs.

### 4-3. Confirmation of the expression of corneal endothelial cell-specific markers by Western blot analysis

A comparison was made of the degree of differentiation into corneal endothelial cells resulting from the direct differentiation of hiPSCs into CECs in the culture media of Examples 3-1 and 3-3.

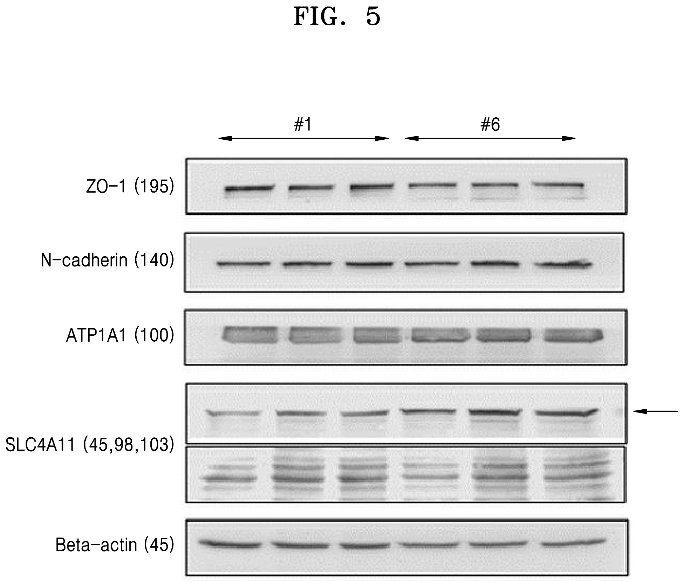

Specifically, CECs directly differentiated from hiPSCs in the culture media of Examples 3-1 and 3-3 were collected, and the protein expression levels of representative corneal endothelial cell-specific markers (ZO-1, N-cadherin, ATP1A1, and SLC4A11) were measured by Western blot. After harvesting the CECs directly differentiated from hiPSCs in the culture media of Examples 3-1 and 3-3, the cells were lysed in RIPA lysis buffer (EPIS BIO, Republic of Korea) containing phosphatase and protease inhibitors (Sigma-Aldrich) according to the manufacturer's protocol. Cell lysates that had been transferred to PVDF membranes (Sigma-Aldrich) using a Trans-Blot-Cell (Bio-Rad Laboratories Inc., Richmond, CA, USA) were separated on 8 % SDS-polyacrylamide gels (SDS-PAGE). Nonspecific protein binding was blocked with 3 % BSA (Sigma-Aldrich) in PBS/0.05 % Tween 20 (PBST). Membranes were incubated overnight with antibodies against ZO-1, N-cadherin, ATP1A1, or SLC4A11 (1:1,000) and β-actin (1:1,000; Cell Signaling Technology, Danvers, MA, USA). After washing, membranes were incubated with horseradish peroxidase-conjugated anti-mouse secondary antibodies (GeneTex, Hsinchu City, Taiwan) at a 1:10,000 dilution. Detection was performed using Gel Doc^{™} XR+ (Bio-Rad Laboratories Inc.) according to the manufacturer's instructions. The results are shown in FIG. 5.

FIG. 5 is a photograph showing the protein expression levels of each corneal endothelial cell-specific marker (ZO-1, N-cadherin, ATP1A1, and SLC4A11) in CECs directly differentiated from iPSCs in the culture media of Examples 3-1 and 3-3, after 12 days of culture, as confirmed by Western blot.

As shown in FIG. 5, SLC4A11 was expressed more strongly in the culture medium of Example 3-3 (arrow), whereas the other corneal endothelial cell markers (ZO-1, N-cadherin, ATP1A1) were expressed at similar levels in both Example 3-1 and Example 3-3.

These results demonstrate that the culture medium of Example 3-3, which lacks ascorbic acid, CaCl₂, and EGF, is as effective for inducing differentiation as the culture medium of Example 3-1.

### Example 5. Comparison of corneal endothelial cell (CEC) groups through single cell sequencing

Single cell sequencing was performed on CECs (ACE2) directly differentiated from iPSCs according to the method of Example 1, CECs (ACE1) differentiated from iPSCs via NCC according to the method of Comparative Example 1, and two types of primary hCECs.

Specifically, the primary hCECs used as controls were cells collected from a 41-year-old female (White/Caucasian) and a 27-year-old female (White/Caucasian), respectively. ACE1 refers to CECs differentiated via NCC according to the method of Comparative Example 1, and ACE2 refers to CECs directly differentiated from iPSCs according to the method of Example 1.

The four samples were classified into 16 gene clusters through single cell sequencing, and the corneal endothelial cell-specific properties were compared.

Specifically, single cell RNA sequencing was performed using the 10X Genomics platform. RNA libraries of the four samples were prepared using Chromium Next GEM Single Cell 3' RNA Library v3.1 (10x Genomics), and 3' digital gene expression profiling was performed for 500 to 10,000 individual cells per sample. The samples were analyzed using Cell Ranger 7.0.1, and cell population analysis was performed using the Seurat 4.3.0 R package.

FIGS. 6A to 6C are graphs showing the results of functional annotation analysis performed through single cell sequencing.

FIG. 6A shows results for biological processes, FIG. 6B shows results for cellular components, and FIG. 6C shows results for molecular functions.

As shown in FIGS. 6A to 6C, compared to the primary hCEC 1 and 2 (human-derived corneal endothelial cell) groups, most cells in the ACE2 group (corneal endothelial cells directly differentiated from induced pluripotent stem cells) also exhibited high levels of expression of corneal endothelial cell genes.

Meanwhile, various corneal endothelial cell-specific gene markers were compared among the hCEC (human-derived corneal endothelial cell) group, the ACE1 (CEC differentiated from iPSCs via NCC) group, and the ACE2 (corneal endothelial cells directly differentiated from induced pluripotent stem cells) group, and the results are shown in FIGS. 7 and 8A and 8B.

FIG. 7 is a heat map comparing the expression patterns of various corneal endothelial cell-specific gene markers among the hCEC (human-derived corneal endothelial cell) group, the ACE1 (CEC differentiated from iPSCs via NCC) group, and the ACE2 (CEC directly differentiated from iPSCs) group.

FIGS. 8A and 8B are graphs analyzing the expression patterns of corneal endothelial cell-specific gene markers (TJP1, ALCAM, ATP1A1, CDH2, COL8A1, and AQP1) in the hCEC (human-derived corneal endothelial cell) group and the ACE2 (corneal endothelial cells directly differentiated from induced pluripotent stem cells) group.

FIG. 8A shows the results of comparing, at the single cell level through uniform manifold approximation and projection (UMAP), the expression of corneal endothelial cell-specific gene markers (TJP1, ALCAM, ATP1A1, CDH2, COL8A1, and AQP1) in the hCEC (human-derived corneal endothelial cell) group and the ACE2 (corneal endothelial cells directly differentiated from induced pluripotent stem cells) group. FIG. 8B is a graph analyzing the expression patterns of corneal endothelial cell-specific gene markers (TJP1, ALCAM, ATP1A1, CDH2, COL8A1, and AQP1) in the hCEC group (blue) and the ACE2 group (orange).

As shown in FIG. 7, the gene cluster expression pattern of the ACE2 group is more similar to that of primary human CECs than is the pattern of the ACE1 group. Furthermore, FIGS. 8A and 8B confirm that the expression patterns of the hCEC group (Cluster 1, left, blue) and the ACE2 group (Cluster 2, right, orange) are highly similar.

These results demonstrate that the corneal endothelial cells (CECs) directly differentiated from induced pluripotent stem cells (iPSCs) by the method according to an aspect of the present disclosure are more similar to human-derived corneal endothelial cells than the CECs differentiated from iPSCs via neural crest cells (NCCs) produced by the conventional method, and furthermore, using the method of direct differentiation from induced pluripotent stem cells (iPSCs) to corneal endothelial cells (CECs) according to an aspect can reduce the required culture time and associated costs.

## Claims

1. A method of differentiating stem cells into corneal endothelial cells (CECs), the method comprising culturing the stem cells in a culture medium comprising a Rho-associated protein kinase (ROCK) inhibitor to differentiate the stem cells into CECs.

2. The method of claim 1,
wherein the culture medium further comprises a TGF-beta inhibitor or insulin-transferrin-selenium (ITS).

3. The method of claim 1,
wherein the culture medium further comprises ascorbic acid, calcium chloride (CaCl₂), or epidermal growth factor (EGF).

4. The method of claim 1,
wherein the ROCK inhibitor comprises one or more selected from the group consisting of fasudil, N-(4-pyridyl)-4β-[(R)-1-aminoethyl]cyclohexane-1α-carboxamide (Y-27632), (2S)-2-methyl-1-[(4-methyl-5-isoquinolinyl)sulfonyl]hexahydro-1H-1,4-diazepine (H-1152), 4-[(1R)-1-aminoethyl]-N-pyridin-4-ylbenzamide hydrochloride (Wf-536), (1R,4r)-4-((R)-1-aminoethyl)-N-(1H-pyrrolo[2,3-b]pyridin-4-yl)cyclohexane-1-carboxamide (Y-30141), N-(3-{[2-(4-amino-1,2,5-oxadiazol-3-yl)-1-ethyl-1H-imidazo[4,5-c]pyridin-6-yl]oxy}phenyl)-4-{[2-(4-morpholinyl)ethyl]oxy}benzamide (GSK269962A), and N-(6-fluoro-1H-indazol-5-yl)-6-methyl-2-oxo-4-[4-(trifluoromethyl)phenyl]-3,4-dihydro-1H-pyridine-5-carboxamide (GSK429286A).

5. The method of claim 1,
wherein the stem cells are embryonic stem cells (ESCs), adult stem cells, or human induced pluripotent stem cells (iPSCs).

6. The method of claim 2,
wherein the TGF-beta inhibitor comprises one or more selected from the group consisting of 4-[4-(2H-1,3-benzodioxol-5-yl)-5-(pyridin-2-yl)-1H-imidazol-2-yl]benzamide (SB431542), 4-(6-(4-(piperazin-1-yl)phenyl)pyrazolo[1,5-a]pyrimidin-3-yl)quinoline (LDN-193189), 4-(2-(6-methylpyridin-2-yl)-5,6-dihydro-4H-pyrrolo[1,2-b]pyrazol-3-yl)quinoline-6-carboxamide (LY-2157299), 4-(2-((4-(2-(pyridin-2-yl)-5,6-dihydro-4H-pyrrolo[1,2-b]pyrazol-3-yl)quinolin-7-yl)oxy)ethyl)morpholine (LY-2109761), 6-(2-(tert-butyl)-4-(6-methylpyridin-2-yl)-1H-imidazol-5-yl)quinoxaline (SB-525334), 2-(3-(6-methylpyridin-2-yl)-1H-pyrazol-4-yl)-1,5-naphthyridine (E-616452), 4-(3-(pyridin-2-yl)-1H-pyrazol-4-yl)quinoline (LY-364947), and 2-(4-((4-((1-cyclopropyl-3-(tetrahydro-2H-pyran-4-yl)-1H-pyrazol-4-yl)oxy)pyridin-2-yl)amino)pyridin-2-yl)propan-2-ol (LY-3200882).

7. The method of claim 1,
wherein the differentiating does not comprise differentiating the stem cells into neural crest cells (NCCs).

8. The method of claim 1,
wherein the method of differentiating comprises culturing in a culture vessel containing the culture medium for 5 to 20 days.

9. A medium composition for inducing direct differentiation of stem
cells into corneal endothelial cells, the composition comprising a Rho-associated protein kinase (ROCK) inhibitor.

10. The medium composition of claim 9,
wherein the medium composition further comprises a TGF-beta inhibitor or insulin-transferrin-selenium (ITS).

11. The medium composition of claim 9,
wherein the medium composition further comprises ascorbic acid, calcium chloride (CaCl₂), or epidermal growth factor (EGF).

12. A kit for inducing direct differentiation of stem cells into corneal
endothelial cells (CECs), the kit comprising: a composition comprising a Rho-associated protein kinase (ROCK) inhibitor; and a culture vessel modified with an extracellular matrix (ECM).

13. The kit of claim 12,
wherein the extracellular matrix comprises on"e or more selected from the group consisting of vitronectin, collagen, fibronectin, laminin, gelatin, matrigel, hyaluronic acid, polylysine, and heparan sulfate proteoglycan.
